# EUROPEAN PATENT SPECIFICATION

(11) **EP 1 938 850 B1**
(45) Date of publication and mention of the grant of the patent: **19.10.2011**
(21) Application number: 07025194.7
(22) Date of filing: 28.12.2007
(51) Int. Cl.: A61M 1/36, A61M 1/02

(54) **Biological fluid filtration systems and methods**
Biologische Fluidfiltriersysteme und Verfahren
Systèmes et procédés de filtration de liquide biologique

(30) Priority: 29.12.2006 US 618286
(43) Date of publication of application: 02.07.2008
(73) Proprietor: Fenwal, Inc., Lake Zurich, IL 60047 (US)
(72) Inventor: Blickhan, Bryan, Zion Illinois 60099 (US); Oleszkiewicz, Anthony, Round Lake Illinois 60037 (US)
(74) Representative: Geary, Stephen

(56) References cited:
- WO-A-96/39940
- DE-A1- 19 537 271
- US-A1- 2003 004 453

## Description

This disclosure generally relates to apparatus and methods for filtering a biological fluid, such as (but not limited to) the removal of leukocytes from whole blood or a blood component. More particularly, the disclosure relates to apparatus and methods for removing air from a fluid container having an amount of filtered fluid.

Prior to transfusion of blood or a blood component into a recipient, it is common to filter the blood to remove leukocytes. This process is commonly referred to as leukoreduction. It may be desirable to remove leukocytes from blood or a blood component prior to transfusion because they can trigger a broad range of adverse reactions in a recipient, ranging from minor effects, such as chills, to more serious effects, such as the transmission of cytomegalovirus, which can be fatal to recipients with weakened immune systems.

Commonly, leukoreduction involves the transfer of blood or a blood component from a pre-filter fluid container to a post-filter fluid container through a tubing line having a leukoreduction filter. The filter typically includes a quantity of air that is pushed out of the filter upon priming the same during a filtration application. It is desired to prevent this air from moving into the post-filter container and remaining there, because such air aggregation can prevent complete filtration of the blood, as will be described in greater detail herein, and decrease the quality and storability of the filtered blood. Even when the blood or blood component is used a short time after filtration, there is a general preference among users to have as little air in the post-filter container as possible. US Patent Application Publication No. 2003/0004453 discloses a bag provided with means for the temporary association of a filter. The bag is intended to receive a biological fluid and comprises an external enclosure of two sheets joined at their periphery and having means disposed on the periphery of the external enclosure for the temporary association of one component and allow the component to be inserted between the means for the temporary association and the external enclosure to provide a temporary association during centrifugation of the bag. A system comprising a bag connected to a filter by means of a tube to allow the filter to be temporarily associated with the bag is also disclosed.

Known approaches to air management include filtration systems that are vented to the atmosphere or a gas container and those incorporating a bypass line. For example, U.S. Patent No. 5,863,436 to Matkovich describes several leukoreduction systems incorporating one or more air vents. One commercial system according to the description of Matkovich is the Pall SAVE™ system, which is incorporated into the Leukotrap® WB Filtration System from Pall Corporation of Glen Cove, NY. The Leukotrap® WB Filtration System comprises a pre-filter container connected to a post-filter container by a filter line having a leukoreduction filter. A pre-filter air vent is associated with the filter line between the pre-filter container and the filter, while a post-filter air vent is associated with the filter line between the filter and the post-filter container. In use, the pre-filter container is hung above the post-filter container and a cannula of the pre-filter container is broken to allow fluid flow into the filter line. The fluid is prevented from flowing into the pre-filter air vent by a removable cap, so it instead flows into the filter. The filter is allowed to prime, with air exiting the system through the post-filter air vent. When the filter is fully primed, a cannula between the post-filter vent and the post-filter container is broken to allow fluid and air to flow into the post-filter container. Due to pressure differentials in the system, the filtration process ceases prior to complete filtration of all the fluid, i.e., with an amount of fluid remaining in the filter. At that time, the cap on the pre-filter air vent is removed to allow a gas to enter the filter line and purge any remaining fluid from the inlet side of the filter.

One problem with systems according to the foregoing description is that no means are provided to remove air from the post-filter container, either during or after filtration. While the post-filter vent removes the air that is purged from the filter, gas may be initially present in the system at other locations, such as in the containers or the tubing, as a result of the manufacturing process. This gas is pushed into the post-filter container during filtration and can lead to the aforementioned diminished performance and quality concerns if not removed during or after filtration.

In response to the foregoing problem, leukoreduction systems incorporating bypass lines allow removal of air and other gases from the post-filter container during and/or after filtration. Several examples of known leukoreduction systems with bypass lines are described in U.S. Patent No. 6,358,420 to Blickhan et al.. In one system, a pre-filter container is connected to a post-filter container by a filter line having a leukoreduction filter. Tubing comprising a bypass line is connected to the filter line at opposite sides of the filter, thereby allowing for fluid communication between the containers along a path that bypasses the filter. The bypass line is provided with a one-way valve, typically a check valve, which only allows air and fluid flow toward the pre-filter container from the post-filter container. In use, the pre-filter container is hung above the post-filter container and a cannula of the pre-filter container is broken to allow fluid flow into the filter line. The fluid is prevented from flowing through the bypass line and into the post-filter container by the one-way valve. The fluid flows through the filter and into the post-filter container, along with an amount of air. Due to pressure differentials in the system, the filtration process ceases prior to complete filtration of the fluid, i.e., with an amount of fluid remaining in the filter. At that time, a slide clamp is placed on the filter line, between the filter and the post-filter container, and the post-filter container is squeezed to force air through the bypass line and toward the pre-filter container. Squeezing the post-filter container to remove air and other gases is sometimes referred to as "burping" the container. When the post-filter container has been "burped," the clamp is removed from the filter line and the filter is allowed to more completely drain.

According to another leukoreduction system described in Blickhan et al., one end of the bypass line is connected to the filter line at a position between the pre-filter container and the filter, while the other end is connected directly to the post-filter container. This system operates similarly to the previously described system of Blickhan et al. to filter blood or a blood component and remove air from the post-filter container.

While systems incorporating bypass lines represent improvements over the systems of Matkovich in terms of air removal from the post-filter container, the need to manually "burp" the container to remove air may be problematic. In particular, the amount of air removal is directly dependent on the skill of the user, which can potentially lead to insufficient or incomplete air removal.

A more recent approach to eliminating the manual "burping" step is to allow for automatic "burping" of the post-filter container. Several such systems are described in U.S. Patent No. 6,171,493 to Zia et al.. Rather than connecting the bypass line to one or more sections of the filter line, one end of the bypass line is directly connected to the pre-filter container and the other end of the bypass line is directly connected to the post-filter container. The pre-filter container is hung above the post-filter container and, in one embodiment, a loop portion of the filter line is elevated above the fluid level in the pre-filter container to prevent fluid from flowing through the bypass line and into the post-filter container. A clamp on the filter line is opened to allow fluid flow through the filter line and the filter. Air in the filter is pushed into the post-filter container by the blood and begins to accumulate therein and/or to leak from the post-filter container into the bypass line. When the pressure in the post-filter container reaches a sufficient level and the pressure in the pre-filter container decreases sufficiently (typically to a vacuum state), some of the air moves up the bypass line, through the loop portion, and into the pre-filter container. The return of air to the pre-filter container increases the pressure above the filter and assists in more completely draining any remaining fluid from the filter.

In theory, the "burping" system of Zia et al. improves on previously known systems by automatically removing air from the post-filter container, without requiring a manual "burping" operation. However, the efficiency of the Zia et al. system is contingent on the pressure differential between the post-filter container and the pre-filter container. Optimal filtration results are achieved when pressure in the post-filter container is maximized. If only a small amount of fluid is to be filtered, then the post-filter container will remain relatively empty and the pressure developed therein will not be sufficient to recirculate the air to the pre-filter container. In such situations, the post-filter container must be manually squeezed to remove air, thereby representing a failure of the intended automatic "burping" feature.

Therefore, there remains a need for apparatus and methods for more efficiently removing air from a post-filter container, especially during filtration of a smaller amount of fluid.

There are several aspects of the present invention which are embodied in the system and method described and claimed below.

In one aspect, a biological fluid filtration system is provided with a pre-filter container adapted to contain a biological fluid, a filter for filtering the biological fluid, and an expandable post-filter container adapted to contain a filtered biological fluid. The pre-filter container and filter are connected by a filter inlet flow path, while the post-filter container and filter are connected by a filter outlet flow path. The system includes a volume restriction cooperatively associated with the post-filter container for limiting the expansion of the post-filter container upon receipt of a biological fluid therein.

In another aspect, a method of filtering a biological fluid includes flowing a biological fluid from a pre-filter container, through a filter, and into an expandable post-filter container. The expansion of the post-filter container upon receipt of biological fluid therein is restrained by a volume restriction upon receipt of a biological fluid therein.

In accordance with a preferred aspect, a method of filtering a biological fluid includes providing a biological fluid filtration system having a pre-filter container adapted to contain a biological fluid, a filter for filtering the biological fluid, and an expandable post-filter container adapted to contain a filtered biological fluid. The system is further provided with a filter inlet flow path extending between the pre-filter container and a filter inlet, and a filter outlet flow path extending between the post-filter container and a filter outlet. The pre-filter container (with a biological fluid) is suspended at a higher vertical elevation than the filter and the post-filter container, and fluid is allowed to flow through the filter and into the post-filter container. Expansion of the post-filter container is restrained upon receipt of biological fluid therein.

Filtration systems and methods generally described herein are particularly well-suited for use in connection with leukoreduction of blood or a blood component. However, filtration systems and methods according to the present invention are not limited to use with specific fluids or filtration processes and may be applied to virtually any biological fluid treatment system involving filtration between two containers.

Figs. 1A-1D are schematic views of a filtration system according to the present invention and a method of using the same;

Figs. 2-5 are schematic views of other filtration systems incorporating a volume restriction;

Fig. 6A is a front elevational view of a post-filter container having a volume restriction provided as a restrictor member, with the container in an unexpanded or pre-filtration condition;

Fig. 6B is a front elevational view of the post-filter container and restrictor member of Fig. 6A, with the container in a restricted maximum volume condition;

Fig. 6C is a side elevational view of the post-filter container and restrictor member of Fig. 6A, with the container in an unexpanded or pre-filtration condition;

Fig. 6D is a side elevational view of the post-filter container and restrictor member of Fig. 6A, with the container in a restricted maximum volume condition;

Fig. 7A is a front elevational view of a post-filter container having an alternative restrictor member, with the container in an unexpanded or pre-filtration condition;

Fig. 7B is a front elevational view of the post-filter container and restrictor member of Fig. 7A, with the container in a restricted maximum volume condition;

Fig. 7C is a side elevational view of the post-filter container and restrictor member of Fig. 7A, with the container in an unexpanded or pre-filtration condition;

Fig. 7D is a side elevational view of the post-filter container and restrictor member of Fig. 7A, with the container in a restricted maximum volume condition;

Fig. 8A is a front perspective view of a post-filter container and a volume restriction provided as a housing;

Fig. 8B is a side cross-sectional view of a housing having a "wedge-shaped" cavity;

Fig. 8C is a side cross-sectional view of the housing of Fig. 8B, with a post-filter container partially received within the cavity;

Fig. 8D is a front perspective view of a housing having a plurality of container-receiving cavities;

Fig. 9A is a front elevational view of a post-filter container having a volume restriction provided as first and second plates;

Fig. 9B is a side elevational view of the post-filter container and plates of Fig. 9A;

Fig. 10A is a front perspective view of a volume restriction provided as a member having a plurality of external radial slots;

Fig. 10B is a top plan view of the member of Fig. 10A, with a plurality of post-filter containers received by the slots;

Fig. 11A is a front elevational view of a post-filter container having a volume restriction provided as an external clamp;

Fig. 11B is a side elevational view of the post-filter container and clamp of Fig. 11A;

Fig. 12A is a front elevational view of a post-filter container in an original condition and a deformed condition;

Fig. 12B is a front elevational view of the post-filter container of Fig. 12A, with the deformed condition maintained by an external clamp;

Figs. 13A-13D are front elevational views of various post-filter containers having a volume restriction provided as an internal bond; and

Fig. 14 is a front elevational view of a post-filter container having a volume restriction provided as a plurality of internal bonds.

The embodiments disclosed herein are for the purpose of providing the required description of the present invention. These embodiments, however, are exemplary of the invention, which may be embodied in various forms. Therefore, specific details disclosed herein are not to be interpreted as limiting the invention as defined in the accompanying claims.

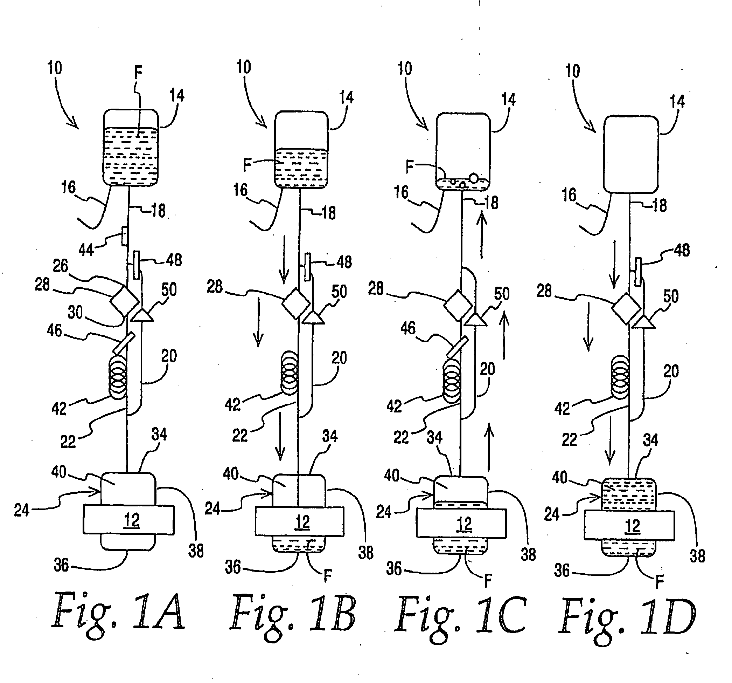

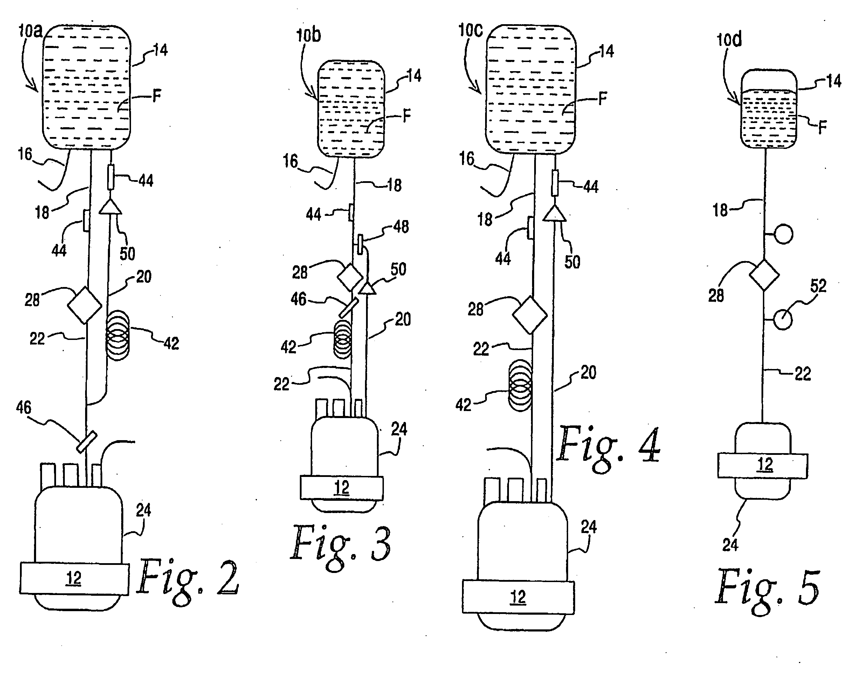

Figs. 1A-1D illustrate a filtration system, generally indicated at 10, suitable for use in combination with a volume restriction 12 (shown schematically in Figs. 1A-1D) of the present invention. Other suitable filtration systems 10a, 10b, 10c, and 10d are illustrated in Figs. 2-5, respectively, although it will be appreciated from the following description that the volume restriction 12 may be used with a wide range of filtration systems, including those systems comprising a component of a larger fluid processing set, and the present invention is not limited to the illustrated systems.

The filtration system 10 of Figs. 1A-1D includes a pre-filter container 14 adapted to contain a biological fluid "F." The pre-filter container 14 may include a needle and associated tubing 16 for drawing an amount of fluid into the container 14, although any method of filling the pre-filter container 14 may be used without departing from the scope of the present invention. According to known design, the pre-filter container 14 may be comprised of a flexible, transparent material, such as polyvinyl chloride or other medical grade plastic.

The pre-filter container 14 includes at least one outlet port with an associated length of tubing, referred to herein as a filter inlet flow path 18. The pre-filter container 14 may include other ports connectable by tubing to various peripheral devices, including other fluid containers. In some systems, such as the filtration systems 10a and 10c of Figs. 2 and 4, one of the ports is associated with a bypass line 20 directly or indirectly connectable to a filter outlet flow path 22 (Fig. 2) or a post-filter container 24 (Fig. 4). These configurations and features will be described in greater detail herein. The exact structure of the pre-filter container 14 is not intended to limit the scope of the present invention and may vary from the particular structures described and illustrated herein.

The filter inlet flow path 18 is preferably connected to a bottom portion of the pre-filter container 14 to allow for fluid flow therethrough preferably by gravity. The other end of the filter inlet flow path 18 is connected to a filter inlet 26 of a filter 28. The filter 28 further includes a filter outlet 30, which is connected to an inlet port of an expandable post-filter container 24 by a length of tubing, referred to herein as a filter outlet flow path 22. The filter inlet flow path 18 and the filter outlet flow path 22 are collectively referred to herein as a filter line. A suitable filter media (not shown) is located within the filter so that fluid passing from the filter inlet flow path 18 to the filter outlet flow path 22 is suitably filtered. The exact structure and function of the filter 28 is not intended to limit the scope of the present invention, but a leukoreduction filter is suitable for use in combination with a biological fluid "F" comprising blood or a blood component. For example, the leukoreduction filters in the Sepacell® line from Asahi Kasei Medical Co., Ltd. of Tokyo, Japan are suitable for use with filtration systems according to the present invention.

The post-filter container 24 is adapted to contain a filtered biological fluid "F" and is comprised of a flexible, preferably transparent material, such as polyvinyl chloride or other medical grade plastic. The post-filter container 24 may be comprised of one or more flexible sheets to define a top end 34, a bottom end 36, and a sidewall 38 extending therebetween. The sidewall 38 defines an interior volume or portion 40 that is expandable because of, for example, stretching of the plastic from a minimum volume to a maximum volume by receipt of fluid "F." During storage, transport, and before filtration has commenced (Fig. 1A), the post-filter container 24 is substantially flat and at or near the minimum volume, typically with a nominal amount of air contained therein as a result of the manufacturing process. As the post-filter container 24 is filled with an increasing amount of fluid "F," it will continually expand and increase in volume, up to the unrestricted maximum volume in which the post-filter container 24 generally resembles a pillow or teardrop.

The post-filter container 24 may include a plurality of ports (Figs. 2-4) connectable by tubing to various peripheral devices, including other fluid containers. In some systems, such as the filtration systems 10b and 10c of Figs. 3 and 4, one of the ports is associated with a bypass line 20 connectable to the filter inlet flow path 18 (Fig. 3) or the pre-filter container 14 (Fig. 4).

In accordance with the present invention, the post-filter container 24 also includes a volume restriction, schematically illustrated in Figs. 1A-5 and generally identified as element 12. The volume restriction 12 may take any of a number of forms, as will be described in greater detail herein, and serves the purpose of limiting the volumetric expansion of the post-filter container 24 upon receipt of a fluid. As per the foregoing description, the efficiency of two-bag filtration systems increases as the filled volume of the post-filter container (i.e., the amount of fluid "F" received by the post-filter container 24) approaches the maximum expanded volume, because air is naturally forced out of the container by the presence of the non-gas fluid. Hence, the volume restriction 12 enhances the performance and efficiency of known filtration systems by limiting the post-filter container to a restricted maximum volume that is less than the unrestricted maximum volume. Preferably, the restricted maximum volume is less than the unrestricted maximum volume, but greater than the volume of biological fluid "F" to be filtered, which allows all of the filtered biological fluid "F" to be held within the post-filter container 24. Alternatively, the restricted maximum volume may be less than or equal to the volume of biological fluid "F" to be filtered, which further prevents residual gas from aggregating in the post-filter container 24 and may force some biological fluid "F" to remain in another portion of the filtration system 10, such as one or more tubing segments 42 (Figs. 1A-1D) for testing. Preferably, the volume restriction 12 is stationary or fixed with respect to the post-filter container 24 during the filtration process, i.e., it does not require manipulation to limit the maximum volume of the post-filter container 24.

A method of using a filtration system 10 and volume restriction 12 of the present invention is illustrated in Figs. 1A-1D. The filtration system 10 conforms generally to the foregoing description, with a pre-filter container 14, a filter inlet flow path 18, a filter 28, a filter outlet flow path 22, a post-filter container 24, and a volume restriction 12. Preferably, the system 10 is sterilized prior to use in a filtration procedure, most preferably during the manufacturing process prior to packaging and transport.

Additional components of the illustrated system 10 include a frangible cannula 44 on the filter inlet flow path 18, a filter line clamp or closure device 46, tubing segments 42 of the filter outlet flow path 22, a bypass line 20 joined to the filter inlet flow path 18 and the filter outlet flow path 22 (by a Y-junction, for example), a bypass line clamp or closure device 48, and a one-way valve 50. The tubing segments 42 may be provided if the filtration system 10 is used to process blood or a blood component. The segments 42 store a quantity of filtered fluid apart from the fluid in the post-filter container 24, which stored fluid is generally used for testing prior to use of the fluid in the post-filter container 24. The structure of the segments 42 may vary, but in one embodiment, the segments 42 comprise two- or three-inch tubing portions that are uniquely labeled for each filtration system 10 to ensure traceability. Each segment is sealable and severable from the remainder of the tubing to allow for testing of fluid "F" contained therein prior to transfusion or other use of the filtered fluid "F" in the post-filter container 24. As for the closure devices 48 and one-way valves 50, they may take any of a number of forms, including a slide clamp or hemostat for the closure device 48 and a check valve or hydrophobic element for the one-way valve 50. The selection of these or other closure and valve elements is well within the capabilities of one having ordinary skill in the art.

In use, the pre-filter container 14 is filled with a biological fluid "F" and suspended at a higher vertical elevation than the filter 28 and the post-filter container 24, as shown in Fig. 1A. The filter line clamp 46 is disengaged from the filter outlet flow path 22 and the cannula 44 is broken (Fig. 1B). The biological fluid "F" flows by gravity through the filter inlet flow path 18 and into the filter 28. The bypass line clamp 48 prevents flow of the fluid "F" through the bypass line 20, although the one-way valve 50 or a second break-away cannula (not illustrated) may perform the same function if the system 10 is provided without a bypass line clamp 48. When the fluid "F" has primed the filter 28, the fluid "F" and any air in the filter 28 flows through the filter outlet flow path 22, the tubing segments 42, and into the post-filter container 24. The post-filter container 24 expands as it receives the biological fluid "F" and air from the filter 28, and the pressure therein increases. The volume restriction 12 limits the expansion of the post-filter container 24, which tends to further increase the pressure in the post-filter container 24.

At the same time that the pressure in the post-filter container 24 is increasing, the pressure in the pre-filter container 14 is decreasing -- typically to a vacuum state. When the pressure in the post-filter container 24 is sufficiently greater than the pressure in the pre-filter container 14, the flow of biological fluid "F" through the filter 28 will cease with an amount of fluid "F" remaining in the filter 28, and possibly in the pre-filter container 14 or filter inlet flow path 18 as well. Ideally, this point occurs with substantially all of the fluid "F" in the post-filter container 24, so that only a small amount of fluid "F" must be flushed from the filter 28. When filtration so ceases, the filter line clamp 46 may be reengaged with the filter outlet flow path 22 and the bypass line clamp 48, if provided, is opened. The pressure in the post-filter container 24 automatically forces the air held therein through the bypass line 20 and the one-way valve 50, into the pre-filter container 14, as shown in Fig. 1C. If present, a vacuum state in the collapsed pre-filter container 14 also assists in drawing fluid "F" upward through the bypass line 20. Thus, the use of the volume restriction 12 will automatically purge all or the majority of air remaining in the post-filter container 24, thereby eliminating the manual "burping" step of known systems and effectively turning them into automatic "burping" systems.

When the air has been re-circulated to the pre-filter container 14, the bypass line clamp 48 is reengaged to the bypass line 20 and the filter line clamp 46 is opened (Fig. 1 D). The accumulation of air in the pre-filter container 14 raises the pressure in the filter inlet flow path 18 above the pressure in the filter outlet flow path 22, which allows the remainder of biological fluid "F" to flow through the filter 28. This filtered biological fluid "F" flows into the post-filter container 24 and some remains in the tubing segments 42 to be tested.

After filtration is complete, the filter outlet flow path 22 and/or the segments 42 may be sealed and severed, and the filtered fluid "F" in the post-filter container 24 may be stored, delivered to a recipient, or otherwise processed. For example, if the fluid "F" is whole blood and the filter 28 is a leukoreduction filter, one common post-filtration process is centrifugation of the post-filter container 24. Depending on the nature of the volume restriction 12, it may be removed from the post-filter container 24 or otherwise be deactivated prior to centrifugation, it may remain with the post-filter container 24 throughout centrifugation, or it may be adapted to disengage from the post-filter container 24 or otherwise deactivate during centrifugation.

The filtration systems 10a-10d of Figs. 2-5 operate similarly to the foregoing method, with variations depending on the particular components. For example, the embodiment of Fig. 4 may allow for continuous air removal from the post-filter container 24, rather than a single purging step. After the system 10c is hung substantially vertically, as shown, cannulas 44 on the filter inlet flow path 18 and bypass line 20 are broken or otherwise opened and fluid flow through the filter 28 begins. Flow through the bypass line 20 is prevented by a loop portion or the like (not illustrated) above the fluid level in the pre-filter container 14 or a one-way valve 50. When the pressure differential between the post-filter container 24 and the pre-filter container 14 reaches a sufficient level, the air is automatically re-circulated through the bypass line 20 to the pre-filter container 14, which essentially allows for "walk-away" operation of the system 10c after the cannulas 44 are opened. The volume restriction 12 acts to further increase the pressure in the post-filter container 24, which encourages air removal therefrom and increases the efficiency of the system 10c.

As for the other illustrated systems, the system 10b of Fig. 3 operates generally according to the method shown in Figs. 1A-1D, with one end of the bypass line 20 being joined directly to the post-filter container 24, instead of to the filter outlet flow path 22. The system 10a of Fig. 2 operates by initially closing flow to the post-filter container 24 with a clamp or closure 46. The cannulas 44 on the filter inlet flow path 18 and the bypass line 20 are opened to allow fluid flow through the filter 28. As the filter 28 primes, the air from the filter 28 is directed through the bypass line 20 and the one-way valve 50, to the pre-filter container 14. If provided on the bypass line 20, the segments 42 will also fill with fluid "F" at this time. To prevent the backflow of fluid "F" into the pre-filter container 14, it may be preferred to provide the one-way valve 50 as a hydrophobic filter. At some point, the backflow of air will cease, at which time the clamp 46 on the filter outlet flow path 22 is opened and filtration will proceed until finished, aided by the air previously circulated to the pre-filter container 14. In this embodiment, the volume restriction 12 aids in removing air from the post-filter container 24 that is initially present as a consequence of the manufacturing process.

Finally, the vented system 10d of Fig. 5 operates according to the foregoing description of U.S. Patent No. 5,863,436 to Matkovich, although the post-filter vent 52 may be placed in other locations downstream of the filter 28, including on the post-filter container 24. This system 10d is "non-burpable" and not intended for removing air from the post-filter container 24 after the air from the filter is removed through vent 52. Some of the benefits provided by the volume restriction 12 may not be fully realized when used in combination with a "non-burpable" system, so it is preferred to use the volume restriction 12 with "burpable" systems, however the present invention is not so limited and may also be used with "non-burpable" systems, such as the one illustrated in Fig. 5, and other dual-container biological fluid filtration systems not specifically illustrated or described herein.

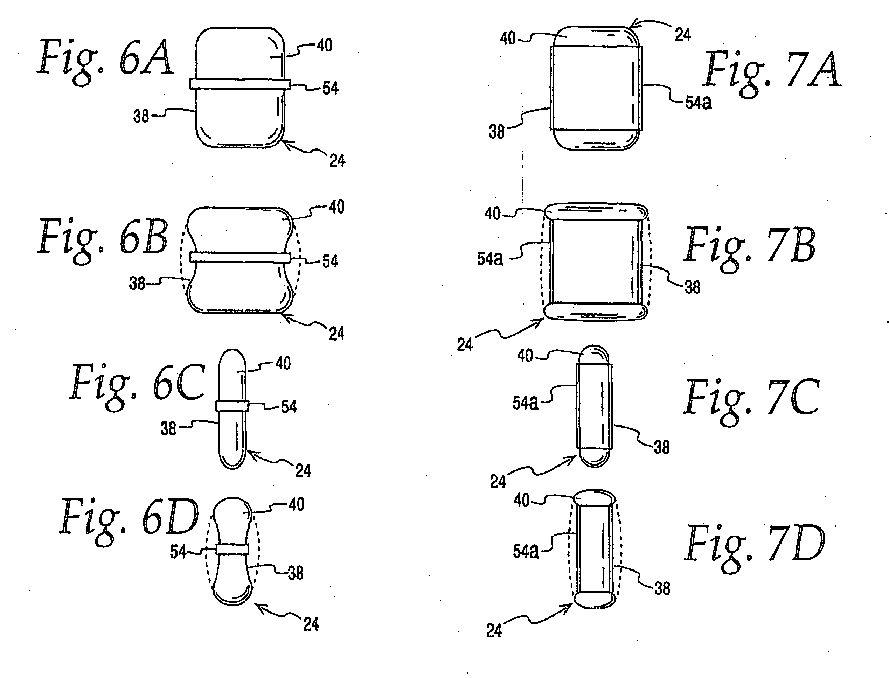

Figs. 6A-14 illustrate a variety of volume restrictions according to the present invention. For example, Figs. 6A-6D show a volume restriction provided as a restrictor member 54. The restrictor member 54 is shown as a belt or band that substantially encircles at least a portion of the post-filter container 24. The post-filter container 24 is expandable from an empty condition (Figs. 6A and 6C) to a restricted maximum volume (Figs. 6B and 6D) that is less than the unrestricted maximum volume, which is shown in broken lines in Figs. 6B and 6D. The restrictor member 54 is substantially non-expandable, or at least less expandable than the post-filter container 24, and acts as an outer boundary for the volumetric expansion of the portion of the container which it surrounds. The placement, size, strength, and inner diameter of the restrictor member 54 determine the restricted maximum volume and may be tailored to the anticipated use of the filtration system. Preferably, the restrictor member 54 is adapted to be used in combination with standard fluid containers, but may also be configured to be used with specially shaped containers.

Numerous variations may be made to the restrictor member 54, such as providing a transparent or semi-transparent restrictor member to allow for improved visibility of the interior of the post-filter container 24. Rather than positioning the restrictor member 54 horizontally, as shown in Figs. 6A-7D), it may be positioned to vertically or diagonally encircle a portion of the post-filter container 24. The restrictor member 54 may be adapted for tightening or loosening to selectively adjust the inner diameter thereof and, in turn, adjust the restricted maximum volume. If a much smaller restricted maximum volume is desired, a relatively wide restrictor member 54a comprising a sleeve (Figs. 7A-7D) or a plurality of restrictor members 54 may be used with the post-filter container 24. The restrictor member 54, 54a may be fixedly secured to the post-filter container 24 or removable therefrom. If secured to the post-filter container 24, any of a number of means may be used, including adhesion, welding, crimping, and the like. If removable from the post-filter container 24, the restrictor member 54, 54a may be elastomeric or have a frangible or weakened zone that is broken to disengage the restrictor member. Alternatively, the restrictor member 54, 54a may be adapted to automatically disengage or at least partially separate from the post-filter container 24 when subjected to the pressures of a centrifuge.

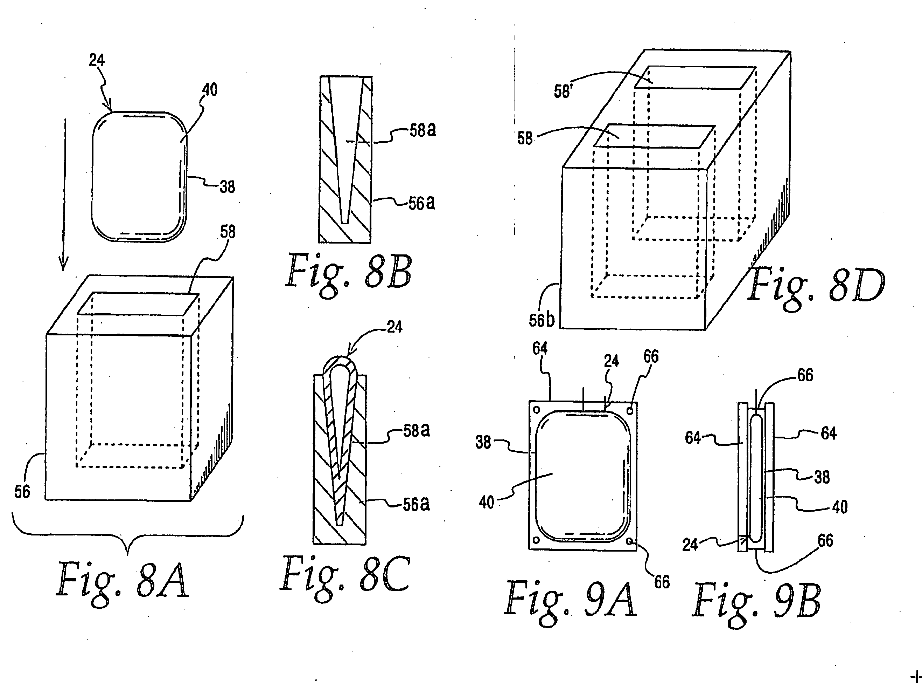

Fig. 8A illustrates a volume restriction provided as a cavity-defining housing 56. The housing 56 is more rigid than the post-filter container 24 and defines a cavity 58 adapted to receive at least a portion of the post-filter container 24. The cavity 58 is sized and configured such that it limits the expansion of the container portion received therein to a restricted maximum volume that is less than the unrestricted maximum volume. The magnitude of the restricted volume may be varied by putting a greater or lesser portion of the post-filter container 24 into the cavity 58. The cavity 58 of Fig. 8A has a generally cuboid shape, although other configuration may be used without departing from the scope of the present invention. For example, Figs. 8B and 8C are cross-sectional views of a housing 56a having a generally "wedge-shaped" cavity 58a, with the perimeter of the cavity 58a decreasing from top to bottom. Such a "wedge-shaped" cavity 58a may be preferred for ease of removing a post-filter container 24 therefrom.

Another variation of the housing is illustrated in Fig. 8D. In this embodiment, the housing 56b includes a second cavity 58', which is adapted to receive a second post-filter container. While the housing 56b is illustrated as having two identical cavities 58 and 58', there may be more than two cavities and/or cavities that are differently shaped, sized, or configured. It will be appreciated that the multiple-cavity housing 56b effectively provides a "volume restriction station" that allows for the simultaneous volume restriction of several post-filter containers, which may be useful for users having a number of filtration applications to carry out.

Figs. 9A and 9B illustrate an embodiment wherein the volume restriction comprises first and second plates 64. A side view of the plates 64 is shown in Fig. 9B, which shows them as being identical and parallel to each other, but they may be differently shaped and/or divergent without departing from the scope of the present invention. The plates 64 are spaced apart from each other to receive at least a portion of a post-filter container 24 therebetween. While the illustrated plates 64 are larger than the post-filter container 24, one or both of the plates 64 may be smaller than the container 24, such that only a portion of the container 24 is received therebetween. As the post-filter container 24 fills with fluid, it bears against the plates 64 and is prevented from expanding to an unrestricted maximum volume. The plates 64 may be fixedly connected to each other by any of a number of connection means, such as the screws 66 shown in Figs. 9A and 9B. In one embodiment, the connection means are adapted to allow for selective adjustment of the separation between the plates 64, which the manufacturer or user may adjust to specify the restricted maximum volume.

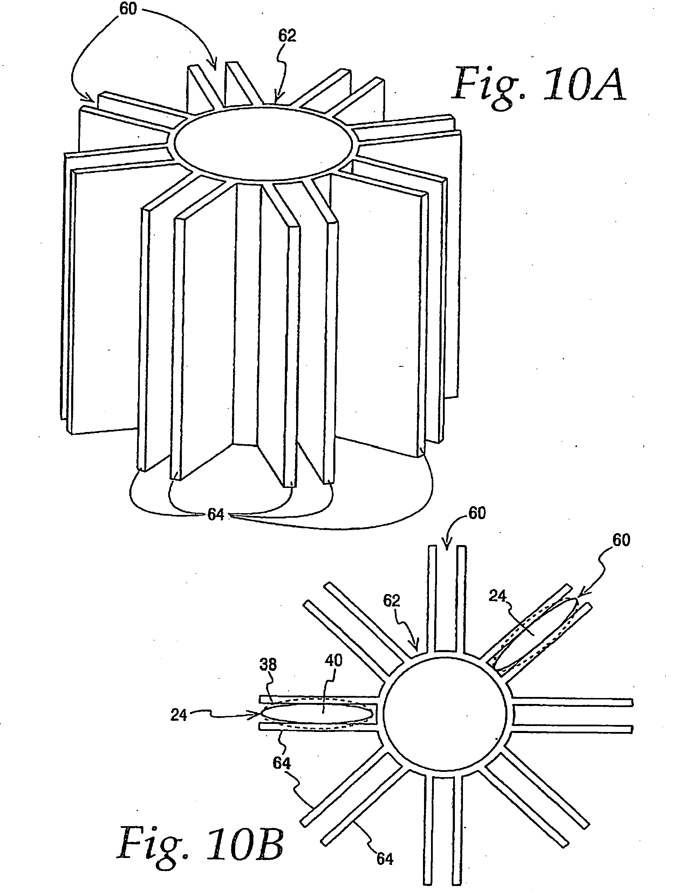

Additional plates may be connected to the first and/or second plates 64 to allow for the simultaneous volume restriction of a plurality of post-filter containers, similar to the "volume restriction station" illustrated in Fig. 8D. Another variation of a "volume restriction station" is illustrated in Figs. 10A and 10B. In this embodiment, the volume restriction comprises a body member 60 having a plurality of plates 64 extending away from the outer surface of the body member 64. The body member 64 is illustrated as a tubular member with radially extending plates 64, but it may be provided in any of a variety of shapes. Adjacent plates define a slot 62 therebetween, which is adapted to receive at least a portion of a post-filter container 24. The shape and size of the slots 62 in the illustrated embodiment depend on the angular separation between adjacent plates and the orientation of adjacent plates (e.g., projecting radially away from the central axis of the body member 60 to form a "wedge-shaped" slot or being parallel to each other, as shown). The illustrated slots 62 are substantially linear and vertical, with a generally uniform width (angular extent) from top to bottom, which maintains a post-filter container 24 received therein in a vertical orientation. However, each slot 62 may be non-linear (e.g., having sinusoidal shapes) and/or non-vertical and may have a varying width along its length. The size and configuration of each slot 62 is sufficiently small to prevent full expansion of the container portion received therein, which limits the container 24 to a restricted maximum volume (Fig. 10B) that is less than the unrestricted maximum volume (shown in broken lines in Fig. 10B).

The volume restrictions of Figs. 8A-10B may be adapted to rest on the ground or other surface or to be suspended above the ground with the post-filter container 24. Preferably, these volume restrictions are adapted to be used in combination with standard fluid containers, but they may also be configured to be used with specially shaped containers. Further, although the housings and plates are illustrated as being solid, they need not be solid and may take a variety of forms, such as mesh-like or other.

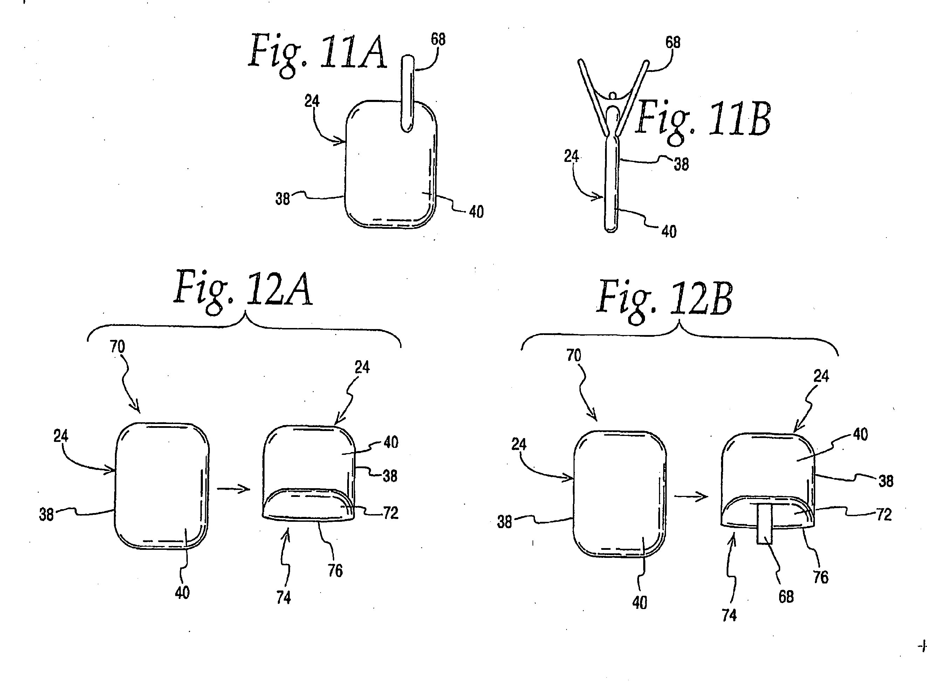

Figs. 11A and 11B illustrate an embodiment of the volume restriction as an external clamp of clip 68. The clamp 68 is illustrated as a typical spring clamp or squeeze clamp, but any squeezing or pinching means (including a one-piece, slide-on "paperclip-like" clip) may also be used without departing from the scope of the present invention. The clamp 68 engages the sidewall 38 of the post-filter container 24 and presses opposing portions of the sidewall 38 against each other in the container interior 40. By action of the clamp 68, the available volume within the interior 40 effectively decreases, as fluid cannot occupy the space between the opposing sidewall portions. Further, it will be appreciated that the clamp 68 prevents or at least limits expansion at and adjacent to the opposing sidewall portions by pressing the opposing portions together. The clamp 68 may be removable from the post-filter container 24, allowing repositioning prior to filtration and/or removal after filtration. Alternatively, a non-removable clamp or a plurality of removable/non-removable clamps may also be used without departing from the scope of the present invention.

Figs. 12A and 12B illustrate an embodiment wherein the volume restriction comprises a deformation of the post-filter container 24. As shown in Fig. 12A, the post-filter container 24 is initially provided in an original condition 70, which is typically substantially flat. A deformed portion 72 is created by deforming the post-filter container 24 from the original condition 70 to a deformed condition 74. The deformed portion 72 of Fig. 12A is formed by creasing and folding a bottom section of the post-filter container 24, so it is referred to herein as a "folded portion," although other deformations, such as tightly rolling a portion of the container, are possible and within the scope of the present invention. The folded portion 72 is separated from the remainder of the container interior by a crease 76, which preferably prevents the inflow of biological fluid into the folded portion 72 during filtration. Hence, it will be appreciated that the available interior volume of the post-filter container 24 is decreased by an amount substantially equal to the volume of the folded portion 72. Similarly, the maximum expanded volume is decreased from an unrestricted maximum expanded volume to a restricted maximum expanded volume, which achieves the benefits previously described herein.

Preferably, the integrity of the folded portion 72 is maintained throughout the filtration process, such that fluid is not allowed into the folded portion 72 during filtration. For example, as shown in Fig. 12B, the folded portion 72 may be enforced by a clamp or clip 68 similar to that illustrated in Figs. 11A and 11B. In another embodiment, the folded portion 72 is adhered or otherwise bonded to the remainder of the post-filter container 24 to prevent a return to the original condition 70 during filtration.

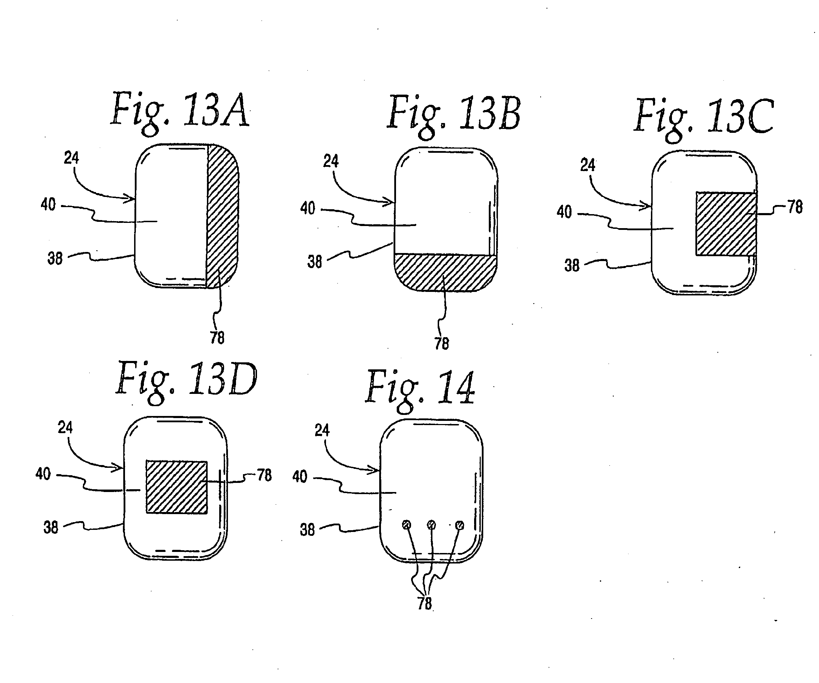

Figs. 13A-14 illustrate embodiments wherein the volume restriction is provided as a bond 78 in the interior 40 of the post-filter container 24. As per the foregoing description, the post-filter container 24 may be comprised of, *inter alia,* a sidewall 38 that defines an open interior portion 40. In the embodiments of Figs. 13A-14, opposing portions of the sidewall 38 are bonded together in the interior portion 40, which limits the interior volume and the maximum expanded volume. The bond 78 may be provided as a surface bond, achieved by an adhesive or surface finish or the like, or a structural bond, achieved by melt-bonding or the like. For example, in one embodiment, opposing portions of the sidewall 38 are treated with a surface finish (such as a heat-activated adhesive) that is adapted to bond the opposing portions together when the post-filter container 24 is sterilized prior to use. Of course, the nature of the bond is dependent on the material of the sidewall 38 and will vary accordingly, so this aspect of the present invention is not limited to a particular bonding process or configuration.

Although the bond 78 is described as being in the interior portion 40 of the post-filter container 24, this aspect of the present invention is not limited to a manufacturing step taking place within the interior portion 40, such as the application of adhesive to the sidewall 38. On the contrary, this aspect of the present invention may include external manipulation that results in a bond 78 in the interior portion 40, such as a melt-bonding process that involves the application of heat to the outside of the sidewall 38, which is then pressed against an opposing portion thereof to establish a structural bond in the interior portion 40.

Figs. 13A-13D illustrate various embodiments of a bond 78 occupying a significant section of the interior portion 40, which will significantly decrease the maximum expanded volume, although the bond 78 is not limited to a specific size or shape. Further, it will be seen from Figs. 13A-13D that the bond 78 may be located in a variety of different positions within the post-filter container 24, but the illustrated configurations are exemplary, rather than limiting, and the bond 78 may be positioned virtually anywhere within the interior portion 40.

Fig. 14 illustrates a plurality of smaller bonds 78 that each joins together highly localized opposing portions of the container sidewall 38. The opposing sections of the sidewall 38 at each bond 78 are held together and at least substantially (if not completely) prevented from expanding upon receipt of a fluid into the post-filter container 24. Similarly, the sections of the container sidewall 38 adjacent to each bond 78 are pulled toward each other, which limits their ability to expand upon receipt of a fluid into the post-filter container 24.

Preferably, the bonds 78 are sufficiently strong that they will not release during filtration. In one embodiment, the bonds are frangible at a force greater than the forces typically present during filtration, which allows at least partial separation of the opposing sidewall portions from each other. For example, a bond may be partially or completely broken to increase the maximum expanded volume before, during, or after filtration. Such a feature may be useful in providing a post-filter container with a relatively large bond that is partially broken by a user prior to filtration according to the amount of fluid to be processed. If the bond is adapted to be broken before or during filtration, it is preferably provided by a bio-compatible adhesive material that will not contaminate the fluid upon contact therewith.

It will be understood that the embodiments described above are illustrative of some of the applications of the principles of the present invention. Numerous modifications may be made by those skilled in the art without departing from the scope of the invention. For these reasons, the scope of the invention is not limited to the above description but is as set forth in the following claims.

## Claims

1. A biological fluid filtration system (10) comprising:
a pre-filter container (14) adapted to contain a biological fluid;
a filter (28) for filtering the biological fluid;
an expandable post-filter container (24) adapted to contain a flltered biological fluid;
a filter inlet flow path (18) extinding between the pre-filter container (14) and a filter inlet (26);
a filter outlet flow path (22) extending between the post-filter container (24) and a filter outlet (30); and
a volume restrictior (12) cooperatively associated with the post-filter container (24) for limiting the expansion of the post-filter container (24) upon receipt of a biological fluid therein.

2. The biological fluid filtration system (10) of claim 1, wherein said volume restriction (12) comprises a restrictor member (54) substantially encircling at least a portion of the post-filter container (24).

3. The biological fluid filtration system (10) of claim 1 or claim 2, wherein said restrictor member (54) includes a frangible zone for breaking and removing the restrictor member (54) from the post-filter container (24).

4. The biological fluid filtration system (10) of claim 2 or claim 3, wherein said restrictor member (54) is substantially comprised of an elastomeric material.

5. The biological fluid filtration system (10) of any one of claims 2 to 4, wherein said restrictor member (54) is adapted to at least partially separate from the post-filter container (24) during centrifugation of the post-filter container (24).

6. The biological fluid filtration system (10) of any one of the preceding claims, wherein said volume restriction (12) comprises a plurality of restrictor members (54) substantially encircling at least a portion of the post-filter container (24)

7. The biological fluid filtration system (10) of any one of the preceding claims, wherein said post-filter container (24) includes an open interior portion (40) defined by a sidewall (38) and said volume restriction (12) comprises a bond between opposing portions of the sidewall (38) in said interior portion (40) to limit the interior volume.

8. The biological fluid filtration system (10) of claim 7, wherein opposing portions of the sidewall (38) include a surface finish adapted to bond the opposing portions together during sterilization of the post-filter container (24).

9. The biological fluid filtration system (10) of claim 7 or claim 8, wherein said bond comprises an area where the opposing portions are melt-bonded together.

10. The biological fluid filtration system (10) of any one of claims 7 to 9, wherein said bond comprises a plurality of separate areas where the opposing portions are bonded together.

11. The biological fluid filtration system (10) of any one of claims 7 to 10, wherein at least a portion of said bond is frangible to allow at least partial separation of the opposing portions from each other.

12. The biological fluid filtration system (10) of any one of the preceding claims, wherein said volume restriction (12) comprises a portion of the post-filter container (24) that is deformed from an original condition to limit biological fluid entering the deformed portion (12) upon receipt of biological fluid into the post-filter container (24).

13. The biological fluid filtration system (10) of claim 12, wherein said deformed portion (12) comprises a folded portion of the post-filter container (24).

14. The biological fluid filtration system (10) of claim 13, wherein at least a portion of the folded portion (72) is bonded to the remainder of the post-filter container (24) to prevent the folded portion (72) from becoming unfolded upon receipt of biological fluid into the post-filter container (24).

15. The biological fluid filtration system (10) of claim 13 or claim 14, wherein at least a portion of the folded portion (72) is clamped to the remainder of the post-filter container (24) to prevent the folded portion (72) from becoming unfolded upon receipt of biological fluid into the post-filter container (24).

16. The biological fluid filtration system (10) of any one of the preceding claims, wherein said volume restriction (12) comprises a relatively rigid housing (56) defining a cavity (58) adapted to receive at least a portion of the post-filter container (24).

17. The biological fluid filtration system (10) of claim 16, wherein said cavity (58) is generally "wedge-shaped."

18. The biological fluid filtration system (10) of claim 16 or claim 17, further comprising a plurality of cavities (58) defined by the housing (56), wherein each cavity (58) is adapted to receive at least a portion of a post-filter container (24).

19. The biological fluid filtration system (10) of any one the preceding claims, wherein said volume restriction (12) comprises first and second plates (64) spaced apart to receive at least a portion of the post-filter container (24) therebetween.

20. The biological fluid filtration system (10) of any one of the preceding claims, wherein said post-flilter container (24) includes an open interior portion (40) defined by a sidewall (38), said volume restriction (12) comprising an external clamp (18) pressing opposing portions of the sidewall (38) together in said interior portion(40).

21. The biological fluid filtration system (10) of claim 20, wherein said clamp (18) is removable from the post-filter container (24).

22. The biological fluid filtration system (10) of any one of the preceding claims, wherein said volume restriction (12) is substantially stationary with respect to the post-rilter container (24) during filtration of a biological fluid.

23. The biological fluid filtration system (10) of any one of the preceding claims, wherein the post-filter contain (24) and the volume restriction (12) are adapted to be suspended at a vertical elevation during filtration of a biological fluid.

24. The biological fluid filtration system (10) of any one of the preceding claims, wherein the volume restriction (17) allows for a restricted maximum volume of the post-filter container (24), wherein the restricted maximum volume is greater than the volume of a biological fluid to be filtered and less than an unrestricted maximum volume of the post-filter container (24).

25. The biological fluid filtration system (10) of any one of the preceding claims, wherein the biological fluid is blood or a blood component.

26. The biological fluid filtration system (10) of claim 25, wherein the filter (28) is a leukoreduction filter.

27. The biological fluid filtration system (10) of any one of the preceding claims, wherein said volume restriction (12) comprises a body member (60) having an outer surface, a plurality of plates (64) extending away from said outer surface, and a plurality of slots (62) defined by adjacent plates (64) wherein each slot (62) is adapted to receive at least a portion of a post-filter container (24) therebetween.

28. A method of filtering a biological fluid, comprising:
flowing a biological fluid from a pre-filter container (14), through a filter (28), and into an expandable post-filter container (24), having a volume restriction (12) associated with the post-filter container (24)
restraining the expansion of the post-filter container (24) using the volume restriction (12) upon receipt of biological fluid in the post filter container(24).

29. The method of claim 28 further comprising:
providing a biological fluid filtration system having
a pre-filter container (14) adapted to contain a biological fluid,
a filter (28) for filtering the biological fluid,
an expandable post-filter container (24) adapted to contain a filtered biological fluid,
a filter inlet flow path (18) extending between the pre-filter container (14) and a filter inlet (26), and
a filter outlet flow path (22) extending between the post-filter container (24) and a filter oulet (30),
suspending the pre-filter container (14) containing a biological fluid at a higher vertical elevation than the filter (28) and the post-filter container (24);
before allowing biological fluid to flow through the filter (28) and into the post-filter container (24), and
restraining the expansion of the post-filter container (24) upon receipt of biological fluid therein.

30. The method of claim 28 or claim 29, further comprising the step of allowing air flow out of the post-filter container (24).

31. The method of any one of claims 28 to 30, wherein said biological fluid is blood or a blood component.

32. The method of claim 31, wherein said allowing a biological fluid to flow through the filter (28) includes removing leukocytes from the blood or blood component

33. The method of any one of claims 28 to 32, wherein said restraining the expansion of the post-filter container (24) includes encircling at least a portion of the post-filter container (24) with a restrictor member (54).

34. The method of any one of claims 28 to 33, wherein said restraining the expansion of the post-filter container (24) providing the post-filter container (24) with an open interior portion (40) defined by a sidewall (38) and bonding together opposing portions of the sidewall (38) in said interior portion (40) to limit the interior volume.

35. The method of claim 34, wherein said restraining the expansion of the post-filter container (24) includes applying a surface finish to the opposing portions of the sidewall (38) and sterilizing the post-filter container (24), thereby bonding the opposing portions together.

36. The method of claim 32 or claim 35 , wherein said restraining the expansion of the post-filter container (24) includes melt-bonding the opposing portions together in said interior portion (40).

37. The method any one of claims 32 to 36, wherein said restraining the expansion of the post-filter container (24) includes bonding the opposing portions together in a plurality of separate areas.

38. The method of any one of claims 28 to 37, wherein said restraining the expansion of the post-filter container (24) includes deforming a portion of the post-filte container (24) from an original condition to-limit biological fluid entering the deformed portion (12) upon receipt of biological fluid into the post-filter container (24).

39. The method of claim 38, wherein said deforming a portion of the post-filter container (24) includes folding a portion of the post-filter container (24) to limit biological fluid entering the folded portion (72) upon receipt of biological fluid into the post-filter container (24).

40. The method of any one of claims 28 to 39, wherein said restraining the expansion of the post-filter container (24) includes positioning at least a portion of the post-filter container (24) in a cavity (58) defined by a relatively rigid housing (56).

41. The method one of claims 28 to 40, wherein said restraining the expansion of the post-filter container (24) includes position at least a portion of the post-filter container between first and second plant (64).

42. The method of any one of claims 28 to 41, wherein said restraining the expansion of the post-filter container (24) includes providing the post-filter container (24) with an interior portion (40) defined by a sidewall (38) and clamping opposing portions of the sidewall (38) together in said interior portion (40).

## Patentansprüche

1. Biologisches Fluidfiltriersystem (10), das Folgendes umfasst:
einen Vorfilterbehälter (14) zum Aufnehmen eines biologischen Fluids;
ein Filter (28) zum Filtern des biologischen Fluids;
einen ausdehnungsfähigen Nachfilterbehälter (24) zum Aufnehmen eines gefilterten biologischen Fluids;
einen Filtereinlass-Strömungsweg (18), der zwischen dem Vorfilterbehälter (14) und einem Filtereinlass (26) verläuft;
einen Filterauslass-Strömungsweg (22), der zwischen dem Nachfilterbehälter (24) und einem Filterauslass (30) verläuft; und
einen Volumenbegrenzer (12), der mit dem Nachfilterbehälter (24) kooperativ assoziiert ist, um die Ausdehnung des Nachfilterbehälters (24) nach der Aufnahme eines biologischen Fluids darin zu begrenzen.

2. Biologisches Fluidfiltriersystem (10) nach Anspruch 1, wobei der genannte Volumenbegrenzer (12) ein Drosselelement (54) umfasst, das wenigstens einen Teil des Nachfilterbehälters (24) im Wesentlichen umschließt.

3. Biologisches Fluidfiltriersystem (10) nach Anspruch 1 oder Anspruch 2, wobei das genannte Drosselelement (54) eine zerbrechliche Zone zum Brechen und Entfernen des Drosselelementes (54) vom Nachfilterbehälter (24) aufweist.

4. Biologisches Fluidfiltriersystem (10) nach Anspruch 2 oder Anspruch 3, wobei das genannte Drosselelement (54) im Wesentlichen aus einem elastomeren Material besteht.

5. Biologisches Fluidfiltriersystem (10) nach einem der Ansprüche 2 bis 4, wobei das genannte Drosselelement (54) so gestaltet ist, dass es sich während des Zentrifugierens des Nachfilterbehälters (24) wenigstens teilweise von dem Nachfilterbehälter (24) löst.

6. Biologisches Fluidfiltriersystem (10) nach einem der vorherigen Ansprüche, wobei die genannte Volumenbegrenzung (12) mehrere Drosselelemente (54) umfasst, die wenigstens einen Teil des Nachfilterbehälters (24) im Wesentlichen umschließen.

7. Biologisches Fluidfiltriersystem (10) nach einem der vorherigen Ansprüche, wobei der genannte Nachfilterbehälter (24) einen durch eine Seitenwand (38) definierten offenen Innenabschnitt (40) aufweist und wobei die genannte Volumenbegrenzung (12) eine Bindung zwischen gegenüberliegenden Abschnitten der Seitenwand (38) in dem genannten Innenabschnitt (40) umfasst, um das Innenvolumen zu begrenzen.

8. Biologisches Fluidfiltriersystem (10) nach Anspruch 7, wobei gegenüberliegende Abschnitte der Seitenwand (38) ein solches Oberflächenfinish aufweisen, dass die gegenüberliegenden Abschnitte beim Sterilisieren des Nachfilterbehälters (24) aneinander gebondet werden.

9. Biologisches Fluidfiltriersystem (10) nach Anspruch 7 oder Anspruch 8, wobei die genannte Bindung einen Bereich umfasst, in dem die gegenüberliegenden Abschnitte miteinander schmelzverbunden werden.

10. Biologisches Fluidfiltriersystem (10) nach einem der Ansprüche 7 bis 9, wobei die genannte Bindung mehrere separate Bereiche umfasst, in denen die gegenüberliegenden Abschnitte aneinander gebondet werden.

11. Biologisches Fluidfiltriersystem (10) nach einem der Ansprüche 7 bis 10, wobei wenigstens ein Abschnitt der genannten Verbindung zerbrechlich ist, so dass die gegenüberliegenden Abschnitte wenigstens teilweise voneinander gelöst werden können.

12. Biologisches Fluidfiltriersystem (10) nach einem der vorherigen Ansprüche, wobei die genannte Volumenbegrenzung (12) einen Abschnitt des Nachfilterbehälters (24) umfasst, der aus einem ursprünglichen Zustand verformt wird, um die Menge an in den verformten Abschnitt (72) eintretendem biologischem Fluid nach der Aufnahme von biologischem Fluid im Nachfilterbehälter (24) zu begrenzen.

13. Biologisches Fluidfiltriersystem (10) nach Anspruch 12, wobei der genannte verformte Abschnitt (72) einen gefalteten Abschnitt des Nachfilterbehälters (24) umfasst.

14. Biologisches Fluidfiltriersystem (10) nach Anspruch 13, wobei wenigstens ein Abschnitt des gefalteten Abschnitts (72) an den Rest des Nachfilterbehälters (24) gebondet wird, um zu verhindern, dass sich der gefaltete Abschnitt (72) nach der Aufnahme von biologischem Fluid im Nachfilterbehälter (24) entfaltet.

15. Biologisches Fluidfiltriersystem (10) nach Anspruch 13 oder Anspruch 14, wobei wenigstens ein Abschnitt des gefalteten Abschnitts (72) an den Rest des Nachfilterbehälters (24) geklemmt wird, um zu verhindern, dass sich der gefaltete Abschnitt (72) nach der Aufnahme von biologischem Fluid im Nachfilterbehälter (24) entfaltet.

16. Biologisches Fluidfiltriersystem (10) nach einem der vorherigen Ansprüche, wobei die genannte Volumenbegrenzung (12) ein relativ starres Gehäuse (56) aufweist, das einen Hohlraum (58) zur Aufnahme von wenigstens einem Abschnitt des Nachfilterbehälters (24) definiert.

17. Biologisches Fluidfiltriersystem (10) nach Anspruch 16, wobei der genannte Hohlraum (58) allgemein "keilförmig" ist.

18. Biologisches Fluidfiltriersystem (10) nach Anspruch 16 oder Anspruch 17, das ferner mehrere vom Gehäuse (56) definierte Hohlräume (58) umfasst, wobei jeder Hohlraum (58) so gestaltet ist, dass er wenigstens einen Abschnitt eines Nachfilterbehälters (24) aufnimmt.

19. Biologisches Fluidfiltriersystem (10) nach einem der vorherigen Ansprüche, wobei die genannte Volumenbegrenzung (12) eine erste und eine zweite Platte (64) umfasst, die beabstandet sind, um wenigstens einen Abschnitt des Nachfilterbehälters (24) dazwischen aufzunehmen.

20. Biologisches Fluidfiltriersystem (10) nach einem der vorherigen Ansprüche, wobei der genannte Nachfilterbehälter (24) einen von einer Seitenwand (38) definierten offenen Innenabschnitt (40) aufweist, wobei die genannte Volumenbegrenzung (12) eine externe Klammer (18) umfasst, die gegenüberliegende Abschnitte der Seitenwand (38) in dem genannten Innenabschnitt (40) zusammenpresst.

21. Biologisches Fluidfiltriersystem (10) nach Anspruch 20, wobei die genannte Klammer (68) vom Nachfilterbehälter (24) entfernt werden kann.

22. Biologisches Fluidfiltriersystem (10) nach einem der vorherigen Ansprüche, wobei die genannte Volumenbegrenzung (12) während des Filterns eines biologischen Fluids in Bezug auf den Nachfilterbehälter (24) im Wesentlichen stationär ist.

23. Biologisches Fluidfiltriersystem (10) nach einem der vorherigen Ansprüche, wobei der Nachfilterbehälter (24) und die Volumenbegrenzung (12) so gestaltet sind, dass sie beim Filtern eines biologischen Fluids in einer vertikalen Höhe aufgehängt werden können.

24. Biologisches Fluidfiltriersystem (10) nach einem der vorherigen Ansprüche, wobei die Volumenbegrenzung (17) ein begrenztes Maximalvolumen des Nachfilterbehälters (24) zulässt, wobei das begrenzte Maximalvolumen größer als das Volumen eines zu filternden biologischen Fluids und kleiner als ein unbegrenztes Maximalvolumen des Nachfilterbehälters (24) ist.

25. Biologisches Fluidfiltriersystem (10) nach einem der vorherigen Ansprüche, wobei das biologische Fluid Blut oder eine Blutkomponente ist.

26. Biologisches Fluidfiltriersystem (10) nach Anspruch 25, wobei das Filter (28) ein Leukoreduktionsfilter ist.

27. Biologisches Fluidfiltriersystem (10) nach einem der vorherigen Ansprüche, wobei die genannte Volumenbegrenzung (12) ein Körperelement (60) mit einer Außenfläche, mehreren von der genannten Außenfläche weg verlaufenden Platten (64) und mehreren durch benachbarte Platten (64) definierten Schlitzen (62) umfasst, wobei jeder Schlitz (62) so gestaltet ist, dass er wenigstens einen Abschnitt eines Nachfilterbehälters (24) dazwischen aufnehmen kann.

28. Verfahren zum Filtern eines biologischen Fluids, das Folgendes beinhaltet:
Fließenlassen eines biologischen Fluids von einem Vorfilterbehälter (14) durch ein Filter (28) und in einen ausdehnungsfähigen Nachfilterbehälter (24) mit einer mit dem Nachfilterbehälter (24) assoziierten Volumenbegrenzung (12),
Begrenzen der Ausdehnung des Nachfilterbehälters (24) mittels der Volumenbegrenzung (12) nach der Aufnahme von biologischem Fluid in dem Nachfilterbehälter (24).

29. Verfahren nach Anspruch 28, das ferner Folgendes beinhaltet:
Bereitstellen eines biologischen Fluidfiltriersystems mit
einem Vorfilterbehälter (14) zum Aufnehmen eines biologischen Fluids,
einem Filter (28) zum Filtern des biologischen Fluids,
einem ausdehnungsfähigen Nachfilterbehälter (24) zum Aufnehmen eines gefilterten biologischen Fluids,
einem Filtereinlass-Strömungsweg (18), der zwischen dem Vorfilterbehälter (14) und einem Filtereinlass (26) verläuft, und
einem Filterauslass-Strömungsweg (22), der zwischen dem Nachfilterbehälter (24) und einem Filterauslass (30) verläuft,
Aufhängen des ein biologisches Fluid enthaltenden Vorfilterbehälters (14) in einer größeren vertikalen Höhe als das Filter (28) und der Nachfilterbehälter (24),
vor dem Fließenlassen des biologischen Fluids durch das Filter (28) und in den Nachfilterbehälter (24), und
Begrenzen der Ausdehnung des Nachfilterbehälters (24) nach der Aufnahme von biologischem Fluid darin.

30. Verfahren nach Anspruch 28 oder Anspruch 29, das ferner den Schritt des Zulassens eines Luftstroms aus dem Nachfilterbehälter (24) beinhaltet.

31. Verfahren nach einem der Ansprüche 28 bis 30, wobei das genannte biologische Fluid Blut oder eine Blutkomponente ist.

32. Verfahren nach Anspruch 31, wobei das genannte Fließenlassen eines biologischen Fluids durch das Filter (28) das Entfernen von Leukocyten aus dem Blut oder der Blutkomponente beinhaltet.

33. Verfahren nach einem der Ansprüche 28 bis 32, wobei das genannte Begrenzen der Ausdehnung des Nachfilterbehälters (24) das Umschließen von wenigstens einem Abschnitt des Nachfilterbehälters (24) mit einem Drosselelement (54) beinhaltet.

34. Verfahren nach einem der Ansprüche 28 bis 33, wobei das genannte Begrenzen der Ausdehnung des Nachfilterbehälters (24) das Versehen des Nachfilterbehälters (24) mit einem durch eine Seitenwand (38) definierten offenen Innenabschnitt (24) und das Aneinanderbonden gegenüberliegender Abschnitte der Seitenwand (38) in dem genannten Innenabschnitt (40) beinhaltet, um das Innenvolumen zu begrenzen.

35. Verfahren nach Anspruch 34, wobei das genannte Begrenzen der Ausdehnung des Nachfilterbehälters (24) das Aufbringen eines Oberflächenfinish auf die gegenüberliegenden Abschnitte der Seitenwand (38) und das Sterilisieren des Nachfilterbehälters (24) beinhaltet, um die gegenüberliegenden Abschnitte aneinander zu bonden.

36. Verfahren nach Anspruch 32 oder Anspruch 35, wobei das genannte Begrenzen der Ausdehnung des Nachfilterbehälters (24) das Schmelzbonden der gegenüberliegenden Abschnitte aneinander in dem genannten Innenabschnitt (40) beinhaltet.

37. Verfahren nach einem der Ansprüche 32 bis 36, wobei das genannte Begrenzen der Ausdehnung des Nachfilterbehälters (24) das Aneinanderbonden der gegenüberliegenden Abschnitte in mehreren separaten Bereichen beinhaltet.

38. Verfahren nach einem der Ansprüche 28 bis 37, wobei das genannte Begrenzen der Ausdehnung des Nachfilterbehälters (24) das Verformen eines Abschnitts des Nachfilterbehälters (24) von einem ursprünglichen Zustand beinhaltet, um den Eintritt von biologischem Fluid in den verformten Abschnitt (72) nach der Aufnahme von biologischem Fluid im Nachfilterbehälter (24) zu begrenzen.

39. Verfahren nach Anspruch 38, wobei das genannte Verformen eines Abschnitts des Nachfilterbehälters (24) das Falten eines Abschnitts des Nachfilterbehälters (24) beinhaltet, um das in den gefalteten Abschnitt (72) eintretende biologische Fluid nach der Aufnahme von biologischem Fluid im Nachfilterbehälter (24) zu begrenzen.

40. Verfahren nach einem der Ansprüche 28 bis 39, wobei das genannte Begrenzen der Ausdehnung des Nachfilterbehälters (24) das Positionieren von wenigstens einem Abschnitt des Nachfilterbehälters (24) in einem durch ein relativ starres Gehäuse (56) definierten Hohlraum (58) beinhaltet.

41. Verfahren nach einem der Ansprüche 28 bis 40, wobei das genannte Begrenzen der Ausdehnung des Nachfilterbehälters (24) das Positionieren von wenigstens einem Abschnitt des Nachfilterbehälters zwischen einer ersten und einer zweiten Platte (64) beinhaltet.

42. Verfahren nach einem der Ansprüche 28 bis 41, wobei das genannte Begrenzen der Ausdehnung des Nachfilterbehälters (24) das Versehen des Nachfilterbehälters (24) mit einem durch eine Seitenwand (58) definierten offenen Innenabschnitt (40) und das Aneinanderklemmen gegenüberliegender Abschnitte der Seitenwand (38) in dem genannten Innenabschnitt (40) beinhaltet.

## Revendications

1. Dispositif de filtration de fluide biologique (10) comprenant :
un conteneur d'avant filtrage (14) adapté afin de contenir un fluide biologique ;
un filtre (28) destiné à filtrer le fluide biologique ;
un conteneur d'après filtrage pouvant être expansé (24) adapté afin de contenir un fluide biologique filtré ;
un trajet d'écoulement d'entrée de filtre (18) s'étendant entre le conteneur d'avant filtrage (14) et une entrée de filtre (26) ;
un trajet d'écoulement de sortie de filtre (22) s'étendant entre le conteneur d'après filtrage (24) et une sortie de filtre (30) ; et
un limiteur de volume (12) associé de manière à coopérer avec le conteneur d'après filtrage (24) afin de limiter l'expansion du conteneur d'après filtrage (24) lors de la réception d'un fluide biologique à l'intérieur.

2. Dispositif de filtration de fluide biologique (10) selon la revendication 1, dans lequel ledit limiteur de volume (12) comprend un élément de limitation (54) entourant sensiblement au moins une partie du conteneur d'après filtrage (24).

3. Dispositif de filtration de fluide biologique (10) selon la revendication 1 ou 2, dans lequel ledit élément de limitation (54) comporte une zone fragile destinée à rompre et à retirer l'élément de limitation (54) du conteneur d'après filtrage (24).

4. Dispositif de filtration de fluide biologique (10) selon la revendication 2 ou 3, dans lequel ledit élément de limitation (54) est constitué principalement en un matériau élastomère.

5. Dispositif de filtration de fluide biologique (10) selon l'une quelconque des revendications 2 à 4, dans lequel ledit élément de limitation (54) est adapté afin de se séparer au moins partiellement du conteneur d'après filtrage (24) au cours de la centrifugation du conteneur d'après filtrage (24).

6. Dispositif de filtration de fluide biologique (10) selon l'une quelconque des revendications précédentes, dans lequel ledit limiteur de volume (12) comprend une pluralité d'éléments de limitation (54) entourant sensiblement au moins une partie du conteneur d'après filtrage (24).

7. Dispositif de filtration de fluide biologique (10) selon l'une quelconque des revendications précédentes, dans lequel ledit conteneur d'après filtrage (24) comporte une partie intérieure ouverte (40) définie par une paroi latérale (38) et ledit limiteur de volume (12) comprend une liaison entre des parties opposées de la paroi latérale (38) dans ladite partie intérieure (40) afin de limiter le volume intérieur.

8. Dispositif de filtration de fluide biologique (10) selon la revendication 7, dans lequel des parties opposées de la paroi latérale (38) comportent une finition de surface adaptée afin de souder les parties opposées ensemble au cours de la stérilisation du conteneur d'après filtrage (24).

9. Dispositif de filtration de fluide biologique (10) selon la revendication 7 ou 8, dans lequel ladite liaison comprend une zone dans laquelle les parties opposées sont soudées ensemble à chaud.

10. Dispositif de filtration de fluide biologique (10) selon l'une quelconque des revendications 7 à 9, dans lequel ladite liaison comprend une pluralité de zones distinctes dans lesquelles les parties opposées sont soudées ensemble.

11. Dispositif de filtration de fluide biologique (10) selon l'une quelconque des revendications 7 à 10, dans lequel au moins une partie de ladite liaison est fragilisée afin de permettre une séparation au moins partielle des parties opposées l'une de l'autre.

12. Dispositif de filtration de fluide biologique (10) selon l'une quelconque des revendications précédentes, dans lequel ledit limiteur de volume (12) comprend une partie du conteneur d'après filtrage (24) qui est déformée à partir d'un état initial afin de limiter l'entrée de fluide biologique dans la partie déformée (72) lors de la réception du fluide biologique dans le conteneur d'après filtrage (24).

13. Dispositif de filtration de fluide biologique (10) selon la revendication 12, dans lequel ladite partie déformée (72) comprend une partie pliée du conteneur d'après filtrage (24).

14. Dispositif de filtration de fluide biologique (10) selon la revendication 13, dans lequel au moins une partie de la partie pliée (72) est soudée au reste du conteneur d'après filtrage (24) afin d'empêcher la partie pliée (72) de se déplier lors de la réception de fluide biologique dans le conteneur d'après filtrage (24).

15. Dispositif de filtration de fluide biologique (10) selon la revendication 13 ou 14, dans lequel au moins une partie de la partie pliée (72) est bloquée sur le reste du conteneur d'après filtrage (24) afin d'empêcher la partie pliée (72) de se déplier lors de la réception de fluide biologique dans le conteneur d'après filtrage (24).

16. Dispositif de filtration de fluide biologique (10) selon l'une quelconque des revendications précédentes, dans lequel ledit limiteur de volume (12) comprend un boîtier relativement rigide (56) définissant une cavité (58) adaptée afin de recevoir au moins une partie du conteneur d'après filtrage (24).

17. Dispositif de filtration de fluide biologique (10) selon la revendication 16, dans lequel ladite cavité (58) est sensiblement en forme de "coin".

18. Dispositif de filtration de fluide biologique (10) selon la revendication 16 ou 17, comprenant, en outre, une pluralité de cavités (58) définies par le boîtier (56), dans lequel chaque cavité (58) est adaptée afin de recevoir au moins une partie d'un conteneur d'après filtrage (24).

19. Dispositif de filtration de fluide biologique (10) selon l'une quelconque des revendications précédentes, dans lequel ledit limiteur de volume (12) comprend des première et seconde plaques (64) espacées l'une de l'autre afin de recevoir au moins une partie du conteneur d'après filtrage (24) entre elles.

20. Dispositif de filtration de fluide biologique (10) selon l'une quelconque des revendications précédentes, dans lequel ledit conteneur d'après filtrage (24) comporte une partie intérieure ouverte (40) définie par une paroi latérale (38), ledit limiteur de volume (12) comprenant une pince externe (68) pressant des parties opposées de la paroi latérale (38) entre elles dans ladite partie intérieure (40).

21. Dispositif de filtration de fluide biologique (10) selon la revendication 20, dans lequel ladite pince (68) est amovible par rapport au conteneur d'après filtrage (24).

22. Dispositif de filtration de fluide biologique (10) selon l'une quelconque des revendications précédentes, dans lequel ledit limiteur de volume (12) est sensiblement fixe par rapport au conteneur d'après filtrage (24) au cours de la filtration d'un fluide biologique.

23. Dispositif de filtration de fluide biologique (10) selon l'une quelconque des revendications précédentes, dans lequel le conteneur d'après filtrage (24) et le limiteur de volume (12) sont adaptés de manière à être suspendus à une certaine élévation verticale au cours de la filtration d'un fluide biologique.

24. Dispositif de filtration de fluide biologique (10) selon l'une quelconque des revendications précédentes, dans lequel le limiteur de volume (12) assure un volume maximum limité du conteneur d'après filtrage (24), dans lequel le volume maximum limité est supérieur au volume d'un fluide biologique à filtrer et inférieur à un volume maximum non limité du conteneur d'après filtrage (24).

25. Dispositif de filtration de fluide biologique (10) selon l'une quelconque des revendications précédentes, dans lequel le fluide biologique est du sang ou un composant sanguin.

26. Dispositif de filtration de fluide biologique (10) selon la revendication 25, dans lequel le filtre (28) est un filtre de réduction de leucocyte.

27. Dispositif de filtration de fluide biologique (10) selon l'une quelconque des revendications précédentes, dans lequel ledit limiteur de volume (12) comprend un élément formant corps (60) présentant une surface externe, une pluralité de plaques (64) s'étendant à partir de ladite surface externe, et une pluralité de fentes (62) définies par des plaques adjacentes (64), dans lequel chaque fente (62) est adaptée de manière à recevoir au moins une partie d'un conteneur d'après filtrage (24).

28. Procédé de filtrage d'un fluide biologique, comprenant :
la mise en circulation d'un fluide biologique à partir d'un conteneur d'avant filtrage (14), à travers un filtre (28), et vers un conteneur d'après filtrage pouvant être expansé (24) comportant un limiteur de volume (12) associé au conteneur d'après filtrage (24) ;
la limitation de l'expansion du conteneur d'après filtrage (24) en utilisant le limiteur de volume (12) lors de la réception du fluide biologique dans le conteneur d'après filtrage (24).

29. Procédé selon la revendication 28, comprenant, en outre:
la préparation d'un dispositif de filtration de fluide biologique comportant :
un conteneur d'avant filtrage (14) adapté afin de contenir un fluide biologique,
un filtre (28) destiné à filtrer le fluide biologique,
un conteneur d'après filtrage pouvant être expansé (24) adapté afin de contenir un fluide biologique filtré,
un trajet d'écoulement d'entrée de filtre (18) s'étendant entre le conteneur d'avant filtrage (14) et une entrée de filtre (26), et
un trajet d'écoulement de sortie de filtre (22) s'étendant entre le conteneur d'après filtrage (24) et une sortie de filtre (30) ;
la mise en suspension du conteneur d'avant filtrage (14) contenant un fluide biologique à une élévation verticale supérieure à celle du filtre (28) et du conteneur d'après filtrage (24) ;
la mise en circulation du fluide biologique à travers le filtre (28) et dans le conteneur d'après filtrage (24) ; et
la limitation de l'expansion du conteneur d'après filtrage (24) lors de la réception de fluide biologique à l'intérieur.

30. Procédé selon la revendication 28 ou 29, comprenant, en outre, l'étape d'autorisation de l'évacuation d'air du conteneur d'après filtrage (24).

31. Procédé selon l'une quelconque des revendications 28 à 30, dans lequel ledit fluide biologique est du sang ou un composant sanguin.

32. Procédé selon revendication 31, dans lequel ladite mise en circulation d'un fluide biologique à travers le filtre (28) comporte l'élimination des leucocytes du sang ou du composant sanguin.

33. Procédé selon l'une quelconque des revendications 28 à 32, dans lequel ladite limitation de l'expansion du conteneur d'après filtrage (24) comporte le cerclage d'au moins une partie du conteneur d'après filtrage (24) avec un élément de limitation (54).

34. Procédé selon l'une quelconque des revendications 28 à 33, dans lequel ladite limitation de l'expansion du conteneur d'après filtrage (24) comporte la préparation du conteneur d'après filtrage (24) avec une partie intérieure ouverte (40) définie par une paroi latérale (38) et la liaison entre elles de parties opposées de la paroi latérale (38) dans ladite partie intérieure (40) afin de limiter le volume intérieur.

35. Procédé selon la revendication 34, dans lequel ladite limitation de l'expansion du conteneur d'après filtrage (24) comporte l'application d'une finition de surface sur les parties opposées de la paroi latérale (38) et la stérilisation du conteneur d'après filtrage (24), soudant ainsi ensemble les parties opposées.

36. Procédé selon la revendication 32 ou 35, dans lequel ladite limitation de l'expansion du conteneur d'après filtrage (24) comporte le soudage à chaud des parties opposées ensemble dans ladite partie intérieure (40).

37. Procédé selon l'une quelconque des revendications 32 à 36, dans lequel ladite limitation de l'expansion du conteneur d'après filtrage (24) comporte le soudage des parties opposées entre elles dans une pluralité de zones séparées.

38. Procédé selon l'une quelconque des revendications 28 à 37, dans lequel ladite limitation de l'expansion du conteneur d'après filtrage (24) comporte la déformation d'une partie du conteneur d'après filtrage (24) à partir d'un état initial afin de limiter l'entrée de fluide biologique dans la partie déformée (72) lors de la réception du fluide biologique dans le conteneur d'après filtrage (24).

39. Procédé selon la revendication 38, dans lequel ladite déformation d'une partie du conteneur d'après filtrage (24) comporte le pliage d'une partie du conteneur d'après filtrage (24) afin de limiter l'entrée de fluide biologique dans la partie pliée (72) lors de la réception de fluide biologique dans le conteneur d'après filtrage (24).

40. Procédé selon l'une quelconque des revendications 28 à 39, dans lequel ladite limitation de l'expansion du conteneur d'après filtrage (24) comporte le positionnement d'au moins une partie du conteneur d'après filtrage (24) dans une cavité (58) définie par un boîtier relativement rigide (56).

41. Procédé selon l'une quelconque des revendications 28 à 40, dans lequel ladite limitation de l'expansion du conteneur d'après filtrage (24) comporte le positionnement d'au moins une partie du conteneur d'après filtrage entre des première et seconde plaques (64).

42. Procédé selon l'une quelconque des revendications 28 à 41, dans lequel ladite limitation de l'expansion du conteneur d'après filtrage (24) comporte l'intégration sur le conteneur d'après filtrage (24) d'une partie intérieure ouverte (40) définie par une paroi latérale (38) et le blocage de parties opposées de la paroi latérale (38) entre elles dans ladite partie intérieure (40).
